(19) Europäisches Patentamt
European Patent Office
Office européen des brevets

(11) Numéro de publication : 0 037 759
B1

(12) **FASCICULE DE BREVET EUROPÉEN**

(45) Date de publication du fascicule du brevet : 05.06.85

(51) Int. Cl.⁴ : A 61 F 2/02, A 61 K 6/00, C 09 J 3/16, C 08 L 63/02, A 61 L 2/08

(21) Numéro de dépôt : 81400421.4

(22) Date de dépôt : 18.03.81

(54) **Ciment utilisable pour la fixation de prothèses osseuses.**

(30) Priorité : 24.03.80 FR 8006479

(43) Date de publication de la demande : 14.10.81 Bulletin 81/41

(45) Mention de la délivrance du brevet : 05.06.85 Bulletin 85/23

(84) Etats contractants désignés : CH DE GB IT LI SE

(56) Documents cités :
DE-A- 1 963 552
DE-A- 2 022 117
FR-A- 1 564 248
FR-A- 2 283 104
GB-A- 952 842
GB-A- 1 050 802
CHEMICAL ABSTRACTS, vol. 90, no. 22, mai 1979, page 59, résumé no. 169797j COLUMBUS, Ohio (US)
CHEMICAL ABSTRACTS, vol. 91, 1979 résumé no. 145930s COLUMBUS, Ohio (US)

(73) Titulaire : COMMISSARIAT A L'ENERGIE ATOMIQUE Etablissement de Caractère Scientifique Technique et Industriel
B.P. 510
F-75752 Paris Cedex 15 (FR)

(72) Inventeur : Le Du, Antoine
10 bis, Allée de Tourville
F-93190 Livry-Gargan (FR)
Inventeur : Michaud, Christian
2, Allée des Genévriers
F-77500 Chelles (FR)
Inventeur : Langlais, Frantz
36, Avenue Reille
F-75014 Paris (FR)
Inventeur : Postel, Michel
2, rue Saint-Simon
F-75007 Paris (FR)

(74) Mandataire : Mongrédien, André et al
c/o BREVATOME 25, rue de Ponthieu
F-75008 Paris (FR)

EP 0 037 759 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention a pour objet un ciment pour la fixation de prothèses osseuses.

On sait que, depuis quelques années, les problèmes posés par le traitement de maladies telles que l'arthrose de la hanche, peuvent être résolus par des interventions chirurgicales qui consistent à supprimer les parties atteintes de l'articulation et à les remplacer par des prothèses métalliques, céramiques ou plastiques afin de retrouver les coefficients de frottement compatibles avec un fonctionnement convenable de l'articulation. Dans le cas de la hanche, les prothèses sont fixées, d'une part, dans le canal médullaire du fémur et, d'autre part, dans la cavité cotyloïdienne du bassin.

Jusqu'à présent, on a réalisé cette fixation au moyen de ciments polymères, obtenus en mélangeant une poudre de polyméthacrylate de méthyle avec du méthacrylate de méthyle monomère, un catalyseur et éventuellement du sulfate de baryum en tant qu'agent opacifiant.

Cependant, de tels ciments ne sont pas totalement satisfaisants du point de vue médical ; en effet, le méthacrylate de méthyle présente une certaine toxicité ; par ailleurs, le durcissement de ces ciments s'effectue avec un dégagement de chaleur trop important, ce qui conduit à des températures relativement élevées de l'ordre de 80 à 90 °C et il se produit de plus un phénomène de retrait lors de la polymérisation.

A ce sujet, il convient de noter que l'emploi de ciments à base de résines synthétiques pose certains problèmes en raison de l'exothermicité des réactions de durcissement et des phénomènes de retrait de la résine lors du durcissement. Aussi, bien que dans d'autres domaines, notamment dans le domaine dentaire, on ait déjà utilisé des ciments à base de résines synthétiques, ceux-ci ne conviennent généralement pas pour la fixation de prothèses osseuses, car les caractéristiques exigées sont différentes pour ces deux utilisations. En effet, pour la fixation de prothèses osseuses, il faut réduire autant que faire se peut le retrait de la résine et il est nécessaire d'utiliser des ciments dont le durcissement ne conduit pas à une réaction trop exothermique afin de limiter l'échauffement d'autant plus que dans ce cas on utilise des quantités plus importantes de ciment que dans le domaine dentaire. Par ailleurs, pour la fixation de prothèses osseuses, il est nécessaire d'utiliser des ciments capables de durcir en milieu humide alors que dans l'art dentaire, le problème ne se pose pas de la même façon puisqu'il est possible d'utiliser des techniques d'assèchement local.

Enfin, pour la fixation de prothèses osseuses, la consistance du ciment a une grande importance pour obtenir une mise en place rapide et sûre du ciment entre les éléments à fixer. De plus, il est avantageux d'obtenir relativement rapidement le durcissement du ciment une fois mis en place.

On connaît toutefois des compositions de ciment à base de résine époxyde, durcissables «*in situ*» qui sont utilisables pour le traitement des fractures osseuses ainsi que pour la réalisation d'attelles, d'éclisses, etc.

Ces compositions décrites dans le brevet anglais GB-A-952 842 comprennent une résine époxyde, de la silice, du silicate d'aluminium, un germicide et un durcisseur à base d'amine, et on peut leur incorporer une autre résine compatible avec la résine époxyde pour les rendre flexibles, par exemple un polymère liquide « thioplaste » ou un copolymère de styrène et de résine alkyde.

Cependant, de telles compositions ne sont pas satisfaisantes pour la fixation de prothèses osseuses car, lors de leur durcissement, on atteint des températures supérieures à 55 °C, comme le montre la fig. 4 où l'élévation de la température atteint 60 °C pour des pièces de 1,5 mm d'épaisseur ou 170 °C pour des pièces de 9,5 mm d'épaisseur. Par ailleurs, elles ne commencent à durcir qu'au bout d'environ 30 min, et leur durcissement n'est achevé qu'au bout de 90 min, ce qui est nettement trop élevé pour une application en chirurgie où l'on doit obtenir le durcissement beaucoup plus rapidement.

La présente invention a précisément pour objet un ciment durcissable à plusieurs composants pour la fixation de prothèses osseuses, qui permet de résoudre les problèmes évoqués ci-dessus.

Le ciment durcissable à plusieurs composants, selon l'invention, comprend un premier composant constitué par un mélange d'une résine assouplissante et d'une résine époxyde présentant une grande réactivité et une toxicité très faible constituée par un diépoxyde du glycidyl éther du bis-phénol A, un deuxième composant constitué par une charge minérale en poudre et un troisième composant constitué par un durcisseur capable de réagir avec la résine époxyde pour provoquer son durcissement, et il se caractérise en ce que la résine assouplissante est une résine réactive et non toxique constituée par un polybutadiène comportant des groupes hydroxyle principalement en bout de chaîne et en ce que le ciment comprend un quatrième composant constitué par un accélérateur de durcissement ; le ciment comprenant pour 100 parties en poids du premier composant, 50 à 85 parties en poids du deuxième composant, 10 à 20 parties en poids du troisième composant et 5 à 10 parties en poids du quatrième composant.

Avantageusement, la résine époxyde et la résine assouplissante ont une viscosité faible, par exemple de l'ordre de 20 Pa.s (200 poises).

De préférence, le mélange de résine époxyde et de résine assouplissante comprend jusqu'à 8 % en poids de résine assouplissante.

Grâce à la présence de ces différents constituants, dans les proportions indiquées ci-dessus, le ciment de l'invention présente les propriétés voulues pour obtenir dans de bonnes conditions la fixation de prothèses osseuses.

Tout d'abord, il présente une consistance parfaitement adaptée à cette utilisation, c'est-à-dire la consistance du mastic, ce qui facilite sa mise en place et le rend compatible avec le protocole opératoire.

Par ailleurs, l'utilisation d'un mélange de résine époxyde et de résine assouplissante réactive permet de modifier et d'adapter les propriétés mécaniques du ciment durci, en particulier d'augmenter sa souplesse, et d'éviter de plus les phénomènes de retrait, ce qui le rend particulièrement adapté à la fixation de prothèses osseuses car dans ce cas le retrait est incompatible. De plus, grâce au choix des différents constituants du mélange, on obtient un faible dégagement de chaleur lors du durcissement du ciment car la réaction n'est pas très exothermique.

Aussi, on peut limiter la température à 55 °C lors du durcissement, ce qui constitue un avantage important pour la fixation de prothèses osseuses étant donné les quantités relativement importantes de résine mises en jeu.

De plus, le ciment selon l'invention présente l'avantage de ne pas être toxique, d'être compatible à long terme avec le milieu biologique, d'être durcissable rapidement (environ 10 min) et d'avoir de bonnes propriétés mécaniques qui restent stables au cours du temps.

Enfin, les composants de ce ciment peuvent être facilement stérilisés sans dégradation, être conditionnés séparément, pendant une longue durée en étant prédosés de façon à faciliter la tâche du chirurgien et éviter toute erreur de dosage au moment de l'utilisation.

Selon l'invention, le choix d'une résine époxyde présentant une grande réactivité et une faible viscosité, de préférence de l'ordre de 20 Pa.s (200 poises), mélangée à une résine assouplissante, permet d'obtenir un mélange très actif qui peut être durci en présence d'eau. Par ailleurs, l'adjonction d'un durcisseur et d'un accélérateur de durcissement appropriés permet d'obtenir un durcissement rapide de ce mélange.

Enfin, le fait d'ajouter une poudre minérale dont la nature chimique et la granulométrie sont choisies de façon à ne pas diminuer la réactivité de la résine, permet d'obtenir après quelques minutes de malaxage des composants, une pâte ayant la consistance du mastic, ne collant pas aux mains, et répondant à la nécessité de pouvoir être mise en place facilement, lors de l'opération.

Selon l'invention, tous les composants du ciment sont choisis de façon à être compatibles avec le milieu biologique et à présenter une toxicité très faible.

Avantageusement, la résine époxyde constituée par un diépoxyde du glycidyl éther du bisphénol A a la formule générale suivante

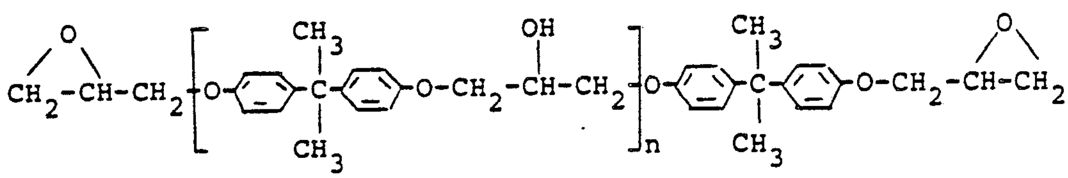

où n est voisin de 0,2.

A titre d'exemple d'une résine de ce type susceptible d'être utilisée, on peut citer la résine époxyde vendue sous la marque « EPIKOTE 828 » par la Société SHELL, qui correspond à la formule ci-dessus et a un poids moléculaire moyen d'environ 380, un équivalent époxy de l'ordre de 190 et un équivalent hydroxy de l'ordre de 1900.

Cette résine présente une bonne réactivité, une faible viscosité (15 Pa.s soit 150 poises) et une faible toxicité.

D'autres résines de la même famille peuvent être utilisées sans que la liste ci-dessous ne soit limitative :

— Araldite 6010 de la Société CIBA
— « Epi-Rez » — Jones Dabney Company (Celanèse Corp.)
— « Epi-Rez » 510 — Jones Dabney Vompany (Celanèse Corp.)
— Epotuf 37-140 — Reichhold Chemical
— ERL 2774 — Union Carbide.

Selon l'invention, la résine assouplissante est un polybutadiène comportant des groupes hydroxyle principalement en bout de chaîne, ayant par exemple la formule suivante :

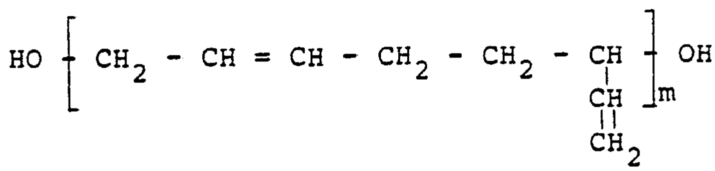

où m a une valeur moyenne voisine de 30 ; par exemple, le produit vendu sous la marque « Butarez HTS » par la Société Philips Petroleum qui présente une bonne réactivité, a une longueur moyenne de chaîne en

poids d'environ 27,7 nm (277 Å), un taux moyen d'hydroxyle en poids de 0,66, une viscosité voisine de 10 Pa.s (100 poises) à 22 °C, et la répartition d'insaturation suivante :

Cis : 34,6 %
Trans : 38,3 %
Vinyle : 27,1 %.

D'autres résines du même type à taux de vinyle égal ou inférieur peuvent également être utilisées.

Selon l'invention, les durcisseurs utilisés sont avantageusement des composés à hydrogène actif, par exemple, des polyamines, des polyphénols, des polyacides, ou des polyalcools. De préférence, on utilise des polyamines telles que la triéthylène tétramine dont la formule est la suivante :

$$NH_2—(CH_2)_2NH—(CH_2)_2—NH—(CH_2)_2—NH_2$$

L'accélérateur de durcissement est choisi de façon à réduire le temps nécessaire pour obtenir le durcissement du mélange et à permettre son durcissement en présence d'eau.

Comme accélérateur de durcissement, on utilise avantageusement, le mercapto-2-éthanol de formule :

$$HS—CH_2—CH_2OH$$

Selon l'invention, la charge minérale en poudre a une granulométrie choisie en fonction de la nature de la résine, en particulier de sa viscosité, pour permettre l'obtention d'un ciment ayant la consistance du mastic au moment de l'utilisation.

Elle est avantageusement constituée par du phosphate de calcium.

Cependant, d'autres charges peuvent être utilisées, par exemple de la silice, de l'alumine, ou encore des fibres courtes de carbone, de silice, etc.

Par ailleurs, on ajoute de préférence à cette charge minérale un agent opacifiant aux rayons X, tel que du sulfate de baryum, ayant une granulométrie du même ordre que celle de la charge minérale.

Enfin, on peut également ajouter au mélange un antibiotique.

A titre d'exemple, un tel mélange peut être constitué par :

— 100 parties en poids de résine époxyde « EPIKOTE 828 »,
— 3,9 parties en poids de polybutadiène (BUTAREZ HTS),
— 13,8 parties en poids de triéthylène tétramine,
— 6,06 parties en poids de mercapto-2-éthanol,
— 65 parties en poids de phosphate de calcium ayant une granulométrie moyenne d'environ 100 µm, et
— 10 à 22 parties en poids de sulfate de baryum.

De préférence, selon l'invention, on conditionne les différents composants de la composition de ciment dans des récipients séparés qui contiennent chacun la dose nécessaire pour le mélange final de façon à faciliter la tâche du chirurgien lors de la préparation du ciment.

Le dispositif de conditionnement du ciment de l'invention comprend une enceinte étanche en matière plastique à l'intérieur de laquelle sont disposés quatre récipients contenant respectivement les doses nécessaires de mélange de résine époxyde et de résine assouplissante, de durcisseur, d'accélérateur et de charge minérale, l'ensemble constitué par l'enceinte contenant les quatre récipients ayant été soumis à une stérilisation.

De préférence, la stérilisation est effectuée par irradiation au moyen de rayonnements ionisants.

Avantageusement, ces récipients sont constitués par trois seringues contenant respectivement le mélange de résines, le durcisseur et l'accélérateur, et un flacon contenant la poudre minérale ; ces trois seringues et le flacon sont placés dans une enceinte étanche constituée par un sac en polyéthylène hermétiquement fermé, qui est ensuite stérilisé au moyen de rayonnements ionisants, par exemple de rayonnements gamma, à une dose d'environ $2,5 \cdot 10^4$ Gy (2,5 Mrad).

D'autres caractéristiques et avantages de l'invention apparaîtront mieux à la lecture de l'exemple suivant donné bien entendu à titre illustratif et non limitatif.

Dans cet exemple, on prépare un ciment à partir des composants suivants :

a) mélange de résine « EPIKOTE 828 » et de résine » BUTAREZ HTS »,
b) durcisseur constitué par de la triéthylène tétramine,
c) accélérateur de durcissement constitué par du mercapto-2-éthanol, et
d) charge minérale constituée par un mélange de phosphate de calcium et de sulfate de baryum.

On conditionne ces différents composants de façon à pouvoir les soumettre ensuite à une stérilisation au moyen de radiations ionisantes.

Dans ce but, on introduit dans une seringue hermétique ayant un volume d'environ 30 ml, réalisée en polypropylène, un mélange de 23,1 g de résine « EPIKOTE 828 » et 0,9 g de « BUTAREZ HTS ».

Dans une seconde et une troisième seringues hermétiques, présentant un volume d'environ 5 ml, réalisées également en polypropylène, on introduit respectivement 3,2 g de triéthylène tétramine et 1,4 g de mercapto-2-éthanol. On remplit un flacon en polyéthylène d'environ 50 ml d'une charge pulvérulente constituée par 15 g de phosphate de calcium ayant une granulométrie moyenne de 100 µm et 2,5 ou 5 g de sulfate de baryum de granulométrie voisine en obturant ensuite le flacon à l'aide d'un bouchon vissé.

On dispose les trois seringues et le flacon dans un sac en polyéthylène avec une pastille de trioxyméthylène, puis on ferme le sac par soudure, et on soumet le tout à une stérilisation à l'aide de rayonnements ionisants à une dose de $2{,}5 \cdot 10^4$ Gy (2,5 Mrad).

On obtient ainsi un sac contenant tous les composants de la composition de ciment qui seront mélangés seulement au moment de leur utilisation.

Lors d'une intervention chirurgicale, la quantité de ciment utilisée est faible et compte tenu de l'importance du temps de polymérisation, il est nécessaire de réaliser le mélange des différents composants dans les conditions d'environnement les plus constantes possibles pour que la dissipation de chaleur lors de la réaction soit toujours la même et n'influe pas sur la durée de cette réaction.

Pour obtenir ce résultat, on utilise des gobelets en polyéthylène et des agitateurs en bois du type « abaisse-langue » classique, qui peuvent avantageusement être disposés dans le sac en polyéthylène et soumis à une stérilisation.

En effet, on a remarqué que l'utilisation de gobelets et d'agitateurs en d'autres matériaux, par exemple, en métaux ou en faïence, conduit à des temps de polymérisation plus longs et moins constants.

Pour effectuer le mélange, on ajoute successivement le mélange de résines, puis la charge et enfin le durcisseur et l'accélérateur ; et on soumet le mélange à un malaxage jusqu'à ce que le produit ait la consistance du mastic.

Dans les conditions habituelles, le mélange durcit en environ 10 min et la température ne dépasse pas 55 °C.

Après durcissement, on note que le retrait volumique est pratiquement nul alors que dans le cas de ciment à base de méthacrylate de méthyle, ce retrait est de l'ordre de 3 %. Par ailleurs, ce ciment présente des propriétés mécaniques satisfaisantes. En effet, lorsqu'on le soumet au bout de deux mois dans l'eau à 37 °C à des essais de traction à une vitesse de 1 mm par min, la contrainte de traction est d'environ 230 bars, ce qui est du même ordre que celle des ciments au méthacrylate essayés dans les mêmes conditions.

Enfin, la conservation des différents composants conditionnés à 5 °C en vue de leur utilisation, c'est-à-dire après introduction dans le sac et stérilisation de l'ensemble, a été testée au bout de 8 mois et on n'a constaté aucune évolution du mélange.

Par ailleurs, les résultats de tests cutanés effectués par la méthode de Magrusson ont montré que les composants du ciment n'étaient ni toxiques, ni allergisants.

## Revendications

1. Ciment durcissable à plusieurs composants, utilisable pour la fixation de prothèses osseuses, comprenant un premier composant constitué par un mélange d'une résine assouplissante et d'au moins une résine époxyde présentant une grande réactivité et une toxicité très faible constituée par un diépoxyde du glicydyléther du bisphénol A, un deuxième composant constitué par une charge minérale en poudre et un troisième composant constitué par un durcisseur capable de réagir avec ladite résine époxyde pour provoquer son durcissement, caractérisé en ce que la résine assouplissante est une résine réactive et non toxique constituée par un polybutadiène comportant des groupes hydroxyle principalement en bout de chaîne, et en ce que le ciment comprend un quatrième composant constitué par un accélérateur de durcissement ;

le ciment comprenant pour 100 parties en poids du premier composant,
50 à 85 parties en poids du deuxième composant,
10 à 20 parties en poids du troisième composant et
5 à 10 parties en poids du quatrième composant.

2. Ciment selon la revendication 1, caractérisé en ce que le mélange de résine époxyde et de résine assouplissante comprend jusqu'à 8 % en poids de résine assouplissante.

3. Ciment selon la revendication 2, caractérisé en ce que le polybutadiène a la formule suivante :

$$HO \left[ CH_2 - CH = CH - CH_2 - CH_2 - \begin{array}{c} CH \\ | \\ CH \\ \| \\ CH_2 \end{array} \right]_m OH$$

où m a une valeur moyenne voisine de 30.

4. Ciment selon l'une quelconque des revendications 1 à 3, caractérisé en ce que la résine époxyde a la formule suivante :

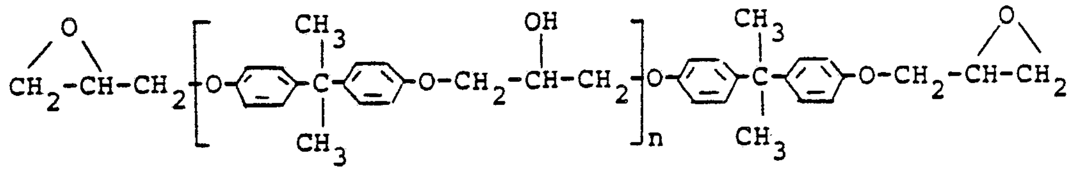

où n est voisin de 0,2.

5. Ciment selon la revendication 4, caractérisé en ce que la résine époxyde a un poids moléculaire moyen d'environ 380, un équivalent époxy de l'ordre de 190 et un équivalent hydroxy de l'ordre de 1900.

6. Ciment selon l'une quelconque des revendications 1 à 5, caractérisé en ce que le durcisseur est une polyamine.

7. Ciment selon la revendication 6, caractérisé en ce que le durcisseur est la triéthylène tétramine.

8. Ciment selon l'une quelconque des revendications 1 à 7, caractérisé en ce que l'accélérateur de durcissement est du mercapto-2-éthanol.

9. Ciment selon l'une quelconque des revendications 1 à 8, caractérisé en ce que la charge minérale est constituée par du phosphate de calcium.

10. Ciment selon l'une quelconque des revendications 1 à 9, caractérisé en ce que la charge minérale comprend un agent opacifiant constitué par du sulfate de baryum.

11. Ciment selon l'une quelconque des revendications 1 à 10, caractérisé en ce qu'il comprend de plus un antibiotique.

12. Ciment selon la revendication 1, caractérisé en ce qu'il comprend, pour 100 parties en poids de résine époxyde de formule :

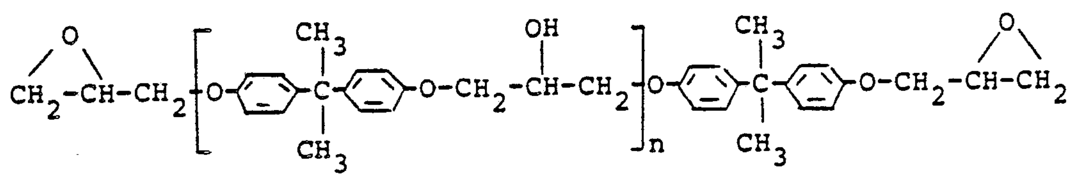

où n est voisin de 0,2.

— 3,9 parties en poids de polybutadiène de formule :

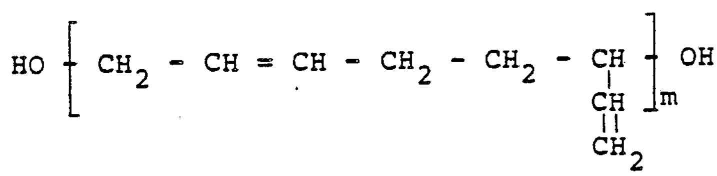

où m a une valeur moyenne voisine de 30,

— 13,8 parties en poids de triéthylène tétramine,

— 6,06 parties en poids de mercapto-2-éthanol,

— 65 parties en poids de phosphate de calcium ayant une granulométrie moyenne de 100 μm, et,

— 10 à 22 parties en poids de sulfate de baryum.

13. Dispositif pour le conditionnement du ciment selon l'une quelconque des revendications 1 à 12, caractérisé en ce qu'il comprend une enceinte étanche en matière plastique à l'intérieur de laquelle sont disposés quatre récipients contenant respectivement les doses nécessaires de mélange de résine époxyde et de résine assouplissante, de durcisseur, d'accélérateur et de charge minérale, l'ensemble constitué par l'enceinte contenant les quatre récipients ayant été soumis à une stérilisation.

14. Dispositif selon la revendication 13, caractérisé en ce que la stérilisation est effectuée par irradiation au moyen de rayonnements ionisants.

**Claims**

1. Multi-component hardenable cement employable for fixation of bone prostheses, comprising a first component constituted by a mixture of a plasticising resin and at least one epoxy resin having high reactivity and very low toxicity, constituted by a diepoxide of the glycidyl ether of bisphenol A, a second component constituted by a powdered mineral filler, and a third component constituted by a hardener adapted to react with said epoxy resin to harden it, characterized in that the plasticising resin is a nontoxic reactive resin constituted by polybutadiene having hydroxyl groups particularly at its chain ends, and in that the cement comprises a fourth component constituted by a hardening accelerator ;

the cement comprising, per 100 parts by weight of first component,

50 to 85 parts by weight of second component,

10 to 20 parts by weight of third component, and

5 to 10 parts by weight of fourth component.

2. Cement according to Claim 1, characterized in that the mixture of epoxy resin and flexibilising resin comprises up to 8 % by weight of the plasticising resin.

3. Cement according to Claim 2, characterized in that the polybutadiene has the formula :

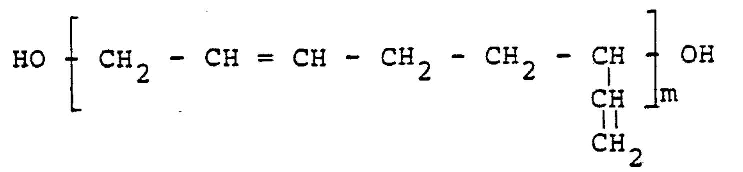

where m has an average value around 30.

4. Cement according to any one of Claims 1 to 3, characterized in that the epoxy resin has the formula :

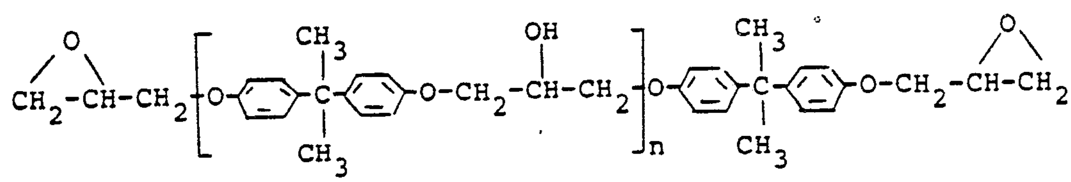

where n is around 0.2.

5. Cement according to Claim 4, characterized in that the epoxy resin has an average molecular weight around 380, an epoxy equivalent of around 190 and a hydroxy equivalent of around 1900.

6. Cement according to any one of Claims 1 to 5, characterized in that the hardener is a polyamine.

7. Cement according to Claim 6, characterized in that the hardener is triethylene tetramine.

8. Cement according to any one of Claims 1 to 7, characterized in that the hardening accelerator is mercapto-2-ethanol.

9. Cement according to any one of Claims 1 to 8, characterized in that the mineral filler comprises calcium phosphate.

10. Cement according to any one of Claims 1 to 9, characterized in that the mineral filler comprises an opacifying agent comprising barium sulphate.

11. Cement according to any one of Claims 1 to 10, characterized in that it additionally comprises an antibiotic.

12. Cement according to Claim 1, characterized in that it comprises, per 100 parts by weight of epoxy resin of the formula :

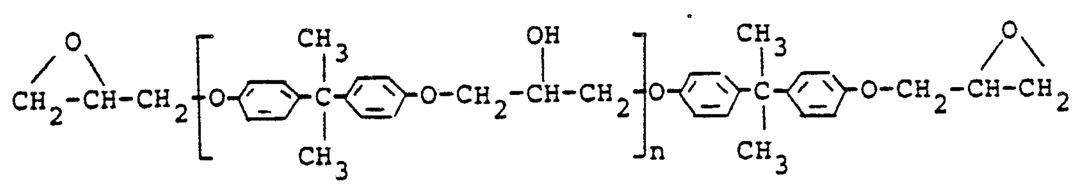

wherein n is around 0.2.

3.9 parts by weight of polybutadiene having the formula :

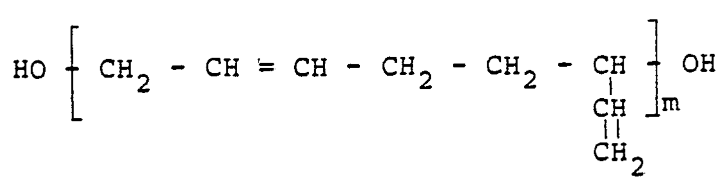

where m has an average value around 30,

13.8 parts by weight of triethylene tetramine,

6.06 parts by weight of mercapto-2-ethanol,

65 parts by weight of calcium phosphate having an average particle size of 100 µm, and

10 to 22 parts by weight of barium sulphate.

13. Apparatus for the production of cement according to any one of Claims 1 to 12, characterized in that it comprises a sealed vessel of plastics material within which are located four containers respectively holding the necessary amounts of the mixture of epoxy resin and plasticising resin, the hardener, the accelerator, and the mineral filler, the assembly comprising the vessel containing the four receivers being sterilized.

14. Apparatus according to Claim 13, characterized in that sterilization is carried out by irradiation with ionizing rays.

**Patentansprüche**

1. Härtbarer Zement aus mehreren Bestandteilen, der für die Verankerung von Knochenprothesen geeignet ist, umfassend einen ersten Bestandteil aus einer Mischung aus einem Weichmacherharz und mindestens einem Epoxidharz, das eine große Reaktionsfähigkeit und eine sehr geringe Toxizität aufweist, bestehend aus einem Diepoxid des Glycidyläthers von Bisphenol A, einen zweiten Bestandteil aus einem pulverförmigen mineralischen Füllstoff und einen dritten Bestandteil aus einem Härter, der mit dem Epoxidharz reagieren kann, um dessen Aushärtung zu bewirken, dadurch gekennzeichnet, daß das Weichmacherharz ein reaktionsfähiges und nicht-toxisches Harz ist, das besteht aus einem Polybutadien, das Hydroxylgruppen hauptsächlich am Ende der Kette trägt, und daß der Zement einen vierten Bestandteil aus einem Härtungsbeschleuniger enthält, wobei

der Zement auf 100 Gew.-Teile des ersten Bestandteils enthält:
50 bis 85 Gew.-Teile des zweiten Bestandteils,
10 bis 20 Gew.-Teile des dritten Bestandteils und
5 bis 10 Gew.-Teile des vierten Bestandteils.

2. Zement nach Anspruch 1, dadurch gekennzeichnet, daß die Epoxidharz/Weichmacherharz-Mischung bis zu 8 Gew.-% Weichmacherharz enthält.

3. Zement nach Anspruch 2, dadurch gekennzeichnet, daß das Polybutadien die folgende Formel hat:

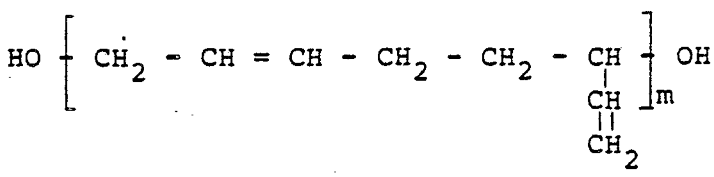

worin m einen mittleren Wert von etwa 30 hat.

4. Zement nach einem der Ansprüche 1 bis 3, dadurch gekennzeichnet, daß das Epoxidharz die folgende Formel hat:

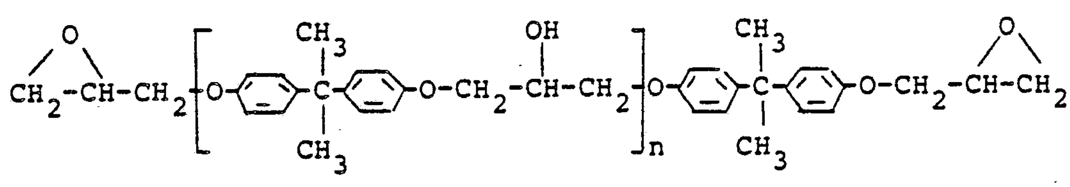

worin n etwa 0,2 beträgt.

5. Zement nach Anspruch 4, dadurch gekennzeichnet, daß das Epoxidharz ein mittleres Molekulargewicht von etwa 380, ein Epoxyäquivalent in der Größenordnung von 190 und ein Hydroxyäquivalent in der Größenordnung von 1900 aufweist.

6. Zement nach einem der Ansprüche 1 bis 5, dadurch gekennzeichnet, daß der Härter ein Polyamin ist.

7. Zement nach Anspruch 6, dadurch gekennzeichnet, daß der Härter Triethylentetramin ist.

8. Zement nach einem der Ansprüche 1 bis 7, dadurch gekennzeichnet, daß der Härtungsbeschleuniger mercapto-2-ethanol ist.

9. Zement nach einem der Ansprüche 1 bis 8, dadurch gekennzeichnet, daß der mineralische Füllstoff aus Calciumphosphat besteht.

10. Zement nach einem der Ansprüche 1 bis 9, dadurch gekennzeichnet, daß der mineralische Füllstoff ein Trübungsmittel enthält, das aus Bariumsulfat besteht.

11. Zement nach einem der Ansprüche 1 bis 10, dadurch gekennzeichnet, daß er außerdem ein Antibioticum enthält.

12. Zement nach Anspruch 1, dadurch gekennzeichnet, daß er enthält auf 100 Gew.-Teile Epoxidharz der Formel:

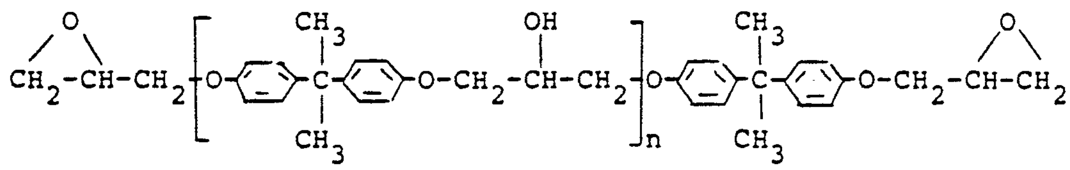

worin n etwa 0,2 bedeutet,

— 3,9 Gew.-Teile Polybutadien der Formel

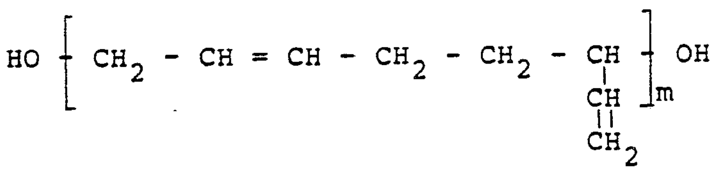

worin m einen mittleren Wert von etwa 30 hat,

— 13,8 Gew.-Teile Triethylentetramin,

— 6,06 Gew.-Teile Mercapto-2-ethanol,

— 65 Gew.-Teile Calciumphosphat mit einer mittleren Korngröße von 100 µm und

— 10 bis 22 Gew.-Teile Bariumsulfat.

13. Vorrichtung zur Konditionierung des Zements nach einem der Ansprüche 1 bis 12, dadurch gekennzeichnet, daß sie umfaßt eine dichte Kammer aus Kunststoff, in deren Innern vier Behälter angeordnet sind, die jeweils die erforderlichen Mengen an Epoxidharz/Weichmacherharz-Mischung, Weichmacher, Beschleuniger und mineralischem Füllstoff enthalten, wobei die Gesamtheit aus der Kammer und den darin enthaltenen vier Behältern einer Sterilisation unterworfen worden ist.

14. Vorrichtung nach Anspruch 13, dadurch gekennzeichnet, daß die Sterilisation durch Bestrahlung mit ionisierenden Strahlen durchgeführt worden ist.